# EUROPÄISCHE PATENTANMELDUNG

(11) **EP 0 634 146 A2**
(43) Veröffentlichungstag der Anmeldung: **18.01.1995**
(21) Anmeldenummer: 94108405.5
(22) Anmeldetag: 01.06.1994
(51) Int. Cl.: A61B 17/32

(54) **Chirurgisches Instrument zum Abtragen von Gewebe**

(30) Priorität: 15.07.1993 DE 4323756
(71) Anmelder: Richard Wolf GmbH, D-75438 Knittlingen (DE)
(72) Erfinder: Heckele, Helmut, D-75438 Knittlingen (DE); Kleih, Jörg, D-72076 Tübingen (DE)
(74) Vertreter: Wilcken, Thomas, Dipl.-Ing.

(57) **Zusammenfassung**

Das Instrument (1) zum Abtragen von Gewebe weist ein am distalen Instrumentenende angeordnetes Werkzeug (2) auf, welches über eine Welle (3) antreibbar ist. Die Welle verläuft durch den mindestens eine Biegestelle (5) aufweisenden Schaft (4) des Instrumentes (1) und ist an jeder Biegestelle (5) des Schaftes (4) unterteilt. Die jeweils benachbarten Enden (6a) der starren Wellenteile (6) stehen über mindestens ein Kreuzgelenk (7, 15) in Verbindung, und jedes Wellenteil (6) ist im Schaft (4) gelagert. Durch die starren Wellenteile (6) und deren Verbindung über Kreuzgelenke (7, 15) sind große Schaftbiegewinkel und die ruckfreie Übertragung großer Drehmomente auf das Werkzeug (2) möglich.

## Beschreibung

Die Erfindung geht aus von einem Instrument zum Abtragen von Gewebe mit einem am distalen Instrumentenende angeordneten Werkzeug, welches über eine Welle antreibbar ist, die durch den starren, mindestens eine Biegestelle aufweisenden Schaft des Instrumentes verläuft.

Für die Chirurgie sind bereits Instrumente dieser Art bekannt, welche insbesondere für die Bereiche in Körperhöhlen gebraucht werden, die auf Grund anatomischer Gegebenheiten mit geraden Instrumenten nicht ohne weiteres zugänglich sind. Deshalb sind derartige Instrumente so konstruiert, daß ihr Schaft am distalen Bereich relativ zu seiner Längsachse abgewinkelt bzw. abgeknickt ist.

Beispielsweise ist aus der DE-OS 38 28 478 ein Resezierinstrument zum Entfernen von Knorpel oder dgl. bekannt, welches ein stirnseitig angeordnetes Schneidenelement aufweist, an das sich ein aus einer Drahtwendel gebildeter Innenschaft anschließt. Um den Innenschaft ist ein zweiteiliger, distalseitig abgewinkelter bzw. abgekröpfter Aussenschaft angeordnet. Der Antrieb des Schneidenelementes bzw. des gewendelten Innenschaftes erfolgt durch einen Motor, der in einem Gehäuse angeordnet ist. Kanäle für Spülflüssigkeit können am Außenrohr angeordnet sein oder auch als bogenförmige Kanäle durch den verbleibenden Freiraum zwischen dem Innenschaft und dem Außenschaft gebildet werden. Da der Innenschaft aus einer elastisch verformbaren Drahtwendel gebildet wird, kann der Außenschaft in Grenzen Abwinkelungen bzw. Biegestellen aufweisen.

Weiterhin ist aus der US-PS 4 646 738 eine Fräse zum Abtragen von Gewebe bekannt. Auch dieses Instrument weist einen elastischen Innenschaft zur Übertragung der Drehbewegung auf ein distalseitig angeordnetes Schneidenelement auf. Abweichend zum zuvor erwähnten Instrument wird der Innenschaft aus drei Wendeln gebildet, die einen unterschiedlichen Wickelsinn aufweisen. Auch hier ist das distale Schaftende im Winkel zur Schaftlängsachse abgekröpft. Eine derartige Konstruktion des Innenschaftes ist jedoch in ihrer Herstellung aufwendig und somit kostenungünstig.

Die EP-OS 0 445 918 beschreibt ein chirurgisches Instrument zur arthroskopischen Anwendung, das aus einem abgewinkelten, starren Außenschaft und einem darin angeordneten und teilweise elastisch verformbaren Innenschaft besteht, dessen proximales Ende mit einem Antrieb gekuppelt ist. Die elastische Verformbarkeit des an sich starren Innenschaftes wird so erreicht, daß der betroffene Schaftabschnitt eine Vielzahl von in geringem Abstand zueinander, wechselseitig zur Schaftlängsachse senkrecht angeordneten Einschnitten aufweist. Ein weiteres motorbetriebenes chirurgisches Instrument für die Arthroskopie ist in der EP-OS 0 481 760 beschrieben, das einen starren Außenschaft mit einem darin angeordneten zweiteiligen Innenschaft aufweist, wobei der distale Schneidenteil und der proximale Antriebsteil mittels einer Art Verzahnung lose miteinander im Eingriff stehen. Ist der Außenschaft distalseitig abgewinkelt, so kann der Innenschaft aus einem elastischen Kunststoff bestehen, so daß keine weiteren konstruktiven Maßnahmen erforderlich sind, um die Drehbewegung auf den distalen Schneidenteil übertragen zu können. Auch diese Lösungen sind aufwendig in ihrer Herstellung und folglich mit hohen Kosten verbunden.

Alle hier aufgeführten Instrumente sind mit einem flexiblen oder zumindest teilweise flexiblen Innenschaft ausgestattet. Der Nachteil einer solchen Ausführung liegt im wesentlichen darin, daß im Bereich der Krümmung eine extrem starke Materialbeanspruchung auftritt. Die laufenden Umformkräfte führen im Material zu frühen Ermüdungserscheinungen und zum Ausfall. Durch Einführung von Einmalgebrauchsinstrumenten wäre dieses Problem nicht gut gelöst, weil sich derartige Instrumente aus ökologischen und ökonomischen Kriterien als ungünstig erweisen. Ein weiterer wesentlicher Nachteil liegt in der sehr begrenzten Abwinkelbarkeit der Instrumente. Zudem arbeiten die flexiblen Elemente nicht wirklich ruckfrei, da eine bauartbedingte Torsionsinstabilität vorliegt.

Durch die Erfindung soll ein demgegenüber stabileres und ruckfrei arbeitendes Instrument vorgeschlagen werden, dessen Schaft erforderlichenfalls auch über einen verhältnismäßig großen Winkel in bezug auf die Längsachse im proximalen Instrumentenbereich abgebogen oder abgeknickt sein kann, ohne daß sich Probleme hinsichtlich der Kraftübertragung mit der Welle ergeben, und das bei hoher Festigkeit gegen Verschleiß im übrigen auch schon alle vorher genannten Anforderungen erfüllt, insbesondere die Übertragung hoher Drehmomente bei gleichzeitig langer Lebensdauer ermöglicht.

Diese Aufgabe wird erfindungsgemäß dadurch gelöst, daß bei dem einleitend erwähnten Instrument die Welle an jeder Biegestelle unterteilt ist, daß die jeweils benachbarten Enden der starren Wellenteile über mindestens ein Kreuzgelenk in Verbindung stehen und daß jedes Wellenteil im Schaft gelagert ist.

Eine vorteilhafte Weiterbildung liegt in der Verwendung eines doppelten Kreuzgelenkes, wodurch bei Bedarf der Winkel jeder Schaftbiegung verhältnismäßig groß ausgeführt werden kann. Die Anzahl der Biegestellen und somit der einzelnen Wellenteile und Kreuzgelenke ist je nach den praktischen Erfordernissen frei zu wählen. Es besteht auch die Möglichkeit, den Schaft in unterschiedliche Richtungen abzuwinkeln bzw. abzukröpfen. Die Wahl von Kreuzgelenken bietet ein ruckfreies Arbeiten. Vorteilhaft ist es auch, daß die Wellenteile starr sind und die Abknickung der Welle nur über die Kreuzgelenke erfolgt. Die Kreuzgelenke geben im Vergleich zu flexiblen Wellenteilen nicht nach, so daß hier keine Materialbelastung auftritt und somit eine hohe Verschleißfestigkeit gewährleistet ist.

Zweckmäßigerweise sind die starren Wellenteile und der zylindrische Endbereich des distal angeordneten Werkzeuges zentriert über Gleitlager im Schaft gelagert. Es besteht auch die Möglichkeit, einen Werkzeugträger für das Werkzeug vorzusehen und diesen zentriert im distalen Schaftende zu lagern. Der Vorteil eines solchen Werkzeugträgers ist, daß mit diesem der Einsatz verschiedener Werkzeuge, wie beispielsweise Messer-, Fräs- oder Bohrkopf, erheblich erleichtert wird, da ein Auswechseln in unkomplizierter Weise erfolgen kann.

Um im übrigen einen Kanal zum Absaugen von Flüssigkeiten oder Partikeln oder aber zum Spülen im Instrument zu integrieren, wird konzentrisch und mit Abstand zum Schaft ein Außenschaft vorgesehen, derart, daß der Raum zwischen den Schäften für einen derartigen Kanal genutzt werden kann. Alternativ hierzu besteht die Möglichkeit, die einzelnen Wellenteile und das Kreuzglenk bzw. die Kreuzglenke innen hohl auszugestalten, so daß ein freier Durchgang für einen zentralen Kanal gegeben ist. In diesem Fall ist es vorteilhaft, eine Rutschkupplung im Bereich des Antriebes vorzusehen, um eine Beschädigung, insbesondere im Bereich des Kreuzgelenkes, zu vermeiden, so daß auch geringe Abmessungen im Kreuzgelenkbereich möglich sind.

Das Kreuzglenk besteht im wesentlichen aus zwei Gelenkgliedern, die die benachbarten Enden der einzelnen starren Wellenteile miteinander verbinden. Alternativ zu dieser Verbindungsart besteht auch die Möglichkeit, die Enden der Wellenteile zu verjüngen und mit Bohrungen zu versehen, so daß direkt Wellenteil und Gelenkglied mittels eines Stiftes miteinander verbindbar sind. Zu diesem Stift ist ein um 90° versetzter weiterer Stift angeordnet, und beide Stifte sind in einer Gelenklasche des Kreuzgelenkes befestigt. Die Stifte bilden senkrecht zueinander stehende Achsen in der Gelenklasche und übertragen hier die von einem proximalen Antrieb ausgehende Drehbewegung über die Wellenteile und das Kreuzgelenk bis hin zum Werkzeug am distalen Instrumentenende.

Eine Ausführung mit einem doppelten Kreuzgelenk kann derart sein, daß die jeweils benachbarten starren Wellenteile über das doppelte Kreuzgelenk, welches aus zwei Einzelgelenken und einem Zwischenglied besteht, mit dem Zwischenglied die jeweilige Biegestelle des Schaftes überbrückt. Eine weitere Möglichkeit ist, daß das doppelte Kreuzgelenk zwei Biegestellen überbrückt, und zwar derart, daß das Zwischenglied in seiner Länge im wesentlichen mit dem Abstand zwischen den beiden Biegestellen übereinstimmt. Eine Biegestelle befindet sich dann jeweils im Bereich eines Einzelgelenkes. Durch eine derartige Ausführung läßt sich ein großer Winkel in bezug auf die Abkröpfung des Schaftes im Verhältnis zur Instrumentenlängsachse realisieren.

Die Erfindung wird nachfolgend anhand von in der Zeichnung dargestellten Ausführungsbeispielen näher erläutert. Es zeigen:
- Figur 1: einen Längsschnitt durch das Instrument mit einer Biegestelle des Schaftes,
- Figuren 2a und 2b: Seitenansichten eines einfachen Kreuzgelenkes zwischen zwei Wellenteilen,
- Figur 3: eine Seitenansicht eines einfachen Kreuzgelenkes mit zentralem Durchlaß,
- Figur 4: einen Schnitt gemäß Linie IV-IV in Figur 3,
- Figuren 5a und 5b: Seitenansichten eines doppelten Kreuzglenkes zwischen zwei Wellenteilen,
- Figur 6: einen Schnitt durch das Instrument im Bereich einer Biegestelle mit einem doppelten Kreuzgelenk und
- Figur 7: einen Schnitt durch das Instrument im Bereich zweier Biegestellen mit einem doppelten Kreuzgelenk.

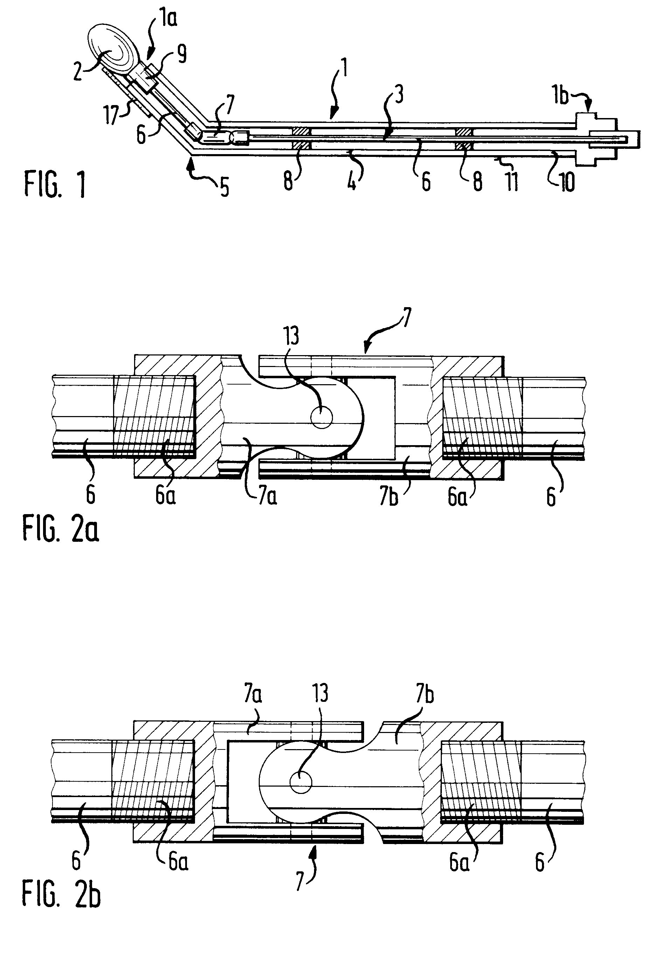

Das Instrument 1 nach Figur 1 besteht aus einem Werkzeug 2 am distalen Instrumentenende 1a, einer das Werkzeug rotierend antreibenden Welle 3 und einem von der Welle durchlaufenen Schaft 4. Das Werkzeug 2 kann ein Messer-, Fräs- oder Bohrkopf sein. Der am proximalen Instrumentenende 1b anzukuppelnde Antrieb kann beispielsweise ein Elektromotor sein, der das Werkzeug 2 über die Welle 3 antreibt.

An der Biegestelle 5 des Schaftes 4 ist die Welle 3 unterteilt, wobei die jeweils benachbarten Enden 6a der starren Wellenteile 6 über ein Kreuzgelenk 7 in Verbindung stehen. Um eine stärkere Abwinkelung des Instrumentes 1 zu erreichen, ist es auch möglich, mehrere Biegestellen 5 sowie entsprechend mehr Wellenteile 6 und Kreuzgelenke 7 im Schaft 4 vorzusehen.

Sowohl die einzelnen Wellenteile 6 als auch der zylindrische Endbereich 9 des Werkzeugs 2 sind zentriert über Gleitlager 8 im Schaft 4 gelagert, wobei der Endbereich 9 direkt das Ende des Werkzeuges 2 oder einen Werkzeugträger darstellen und einen Gleitlagerteil bilden kann. Konzentrisch zum Schaft 4 ist ein Außenschaft 11 angeordnet. Zwischen diesen beiden Schäften verläuft ein Kanal 10, welcher als Spül- und Saugkanal dient. Schließlich zeigt Figur 1 einen am distalen Instrumentenende 1a angeordneten Schutz 17 für das Werkzeug.

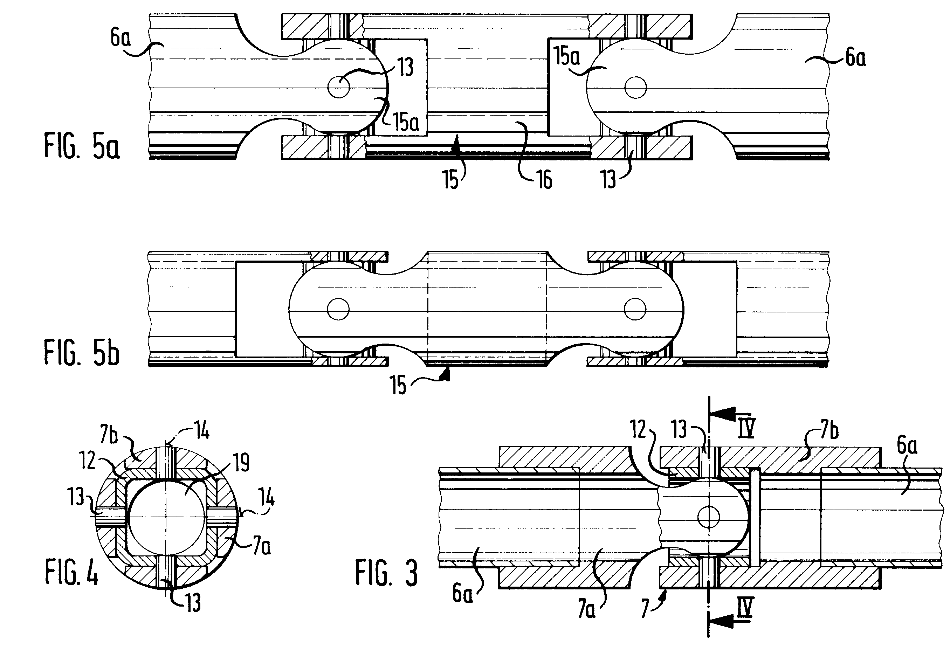

In den Figuren 2 bis 4 ist der Aufbau eines einfachen Kreuzgelenkes 7 zu erkennen, welches die Enden 6a der Wellenteile 6 drehbeweglich miteinander verbindet. Das Kreuzgelenk 7 besteht im wesentlichen aus zwei Gelenkgliedern 7a und 7b, die die Enden 6a der Wellenteile 6 miteinander verbinden. Die Gelenkglieder 7a und 7b werden von einer Gelenklasche 12 (Figur 3) überbrückt, welche die Gelenkglieder 7a und 7b drehbeweglich über um 90° zueinander angeordnete Stifte 13 miteinander verbindet, wobei die Stifte 13 in der Gelenklasche 12 fixiert sind. Die Stifte 13 bilden senkrecht zueinander stehende Lagerachsen 14 in der Gelenklasche 12.

Die Figuren 3 und 4 zeigen ein einfaches Kreuzglenk 7, welches innen hohl ist. Bei dieser Ausführungsform müssen auch die Wellenteile 6 innen hohl sein, wenn durch die Wellenteile und die Durchgänge 19 in den Gelenken Spülflüssigkeit und abgetragene Gewebepartikel durch das Instrument geleitet werden sollen.

Ein weiteres Ausführungsbeispiel zeigen die Figuren 5a und 5b. Hier ist ein doppeltes Kreuzglenk 15 dargestellt, welches aus zwei Einzelgelenken 15a besteht, die über ein Zwischenglied 16 miteinander verbunden sind. Die Enden 6a der Wellenteile 6 sind verjüngt und mit Bohrungen versehen, durch die die Stifte 13 führen. Die Stifte 13 sind auch hier wieder um 90° zueinander versetzt angeordnet und übertragen die Drehbewegung von einem Wellenteil über das Zwischenglied 16 auf das benachbarte Wellenteil.

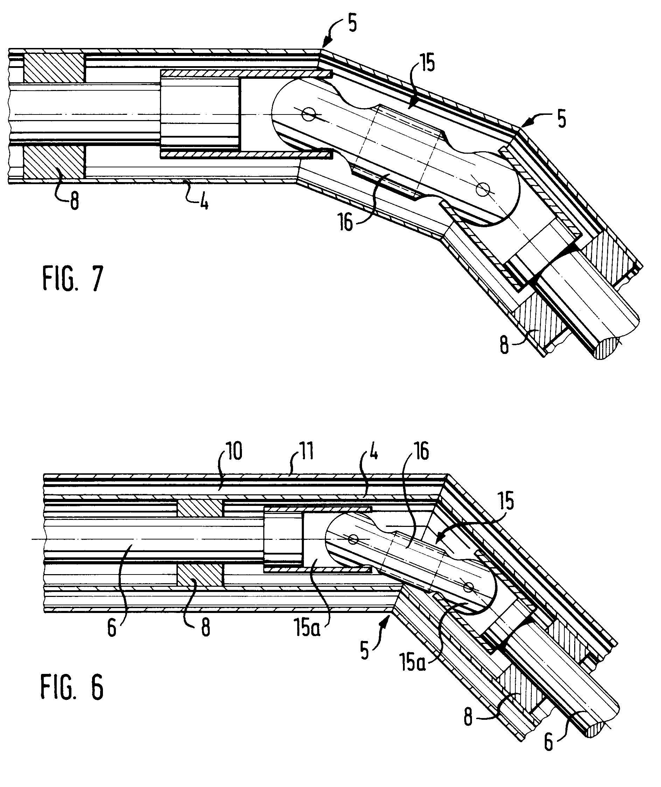

In Figur 6 ist ein doppeltes Kreuzglenk 15 zu erkennen, welches zwei Wellenteile 6 an einer Biegestelle 5 des Schaftes 4 miteinander verbindet. Das Zwischenglied 16 ist im Bereich der Biegestelle 5 angeordnet.

Alternativ zu dieser Anordnung ist als weiteres Ausführungsbeispiel nach Figur 7 ein doppeltes Kreuzgelenk 15 im Schaft 4 derart vorgesehen, daß es zwei Biegestellen 5 des Schaftes 4 überbrückt. Der Abstand der Biegestellen 5 richtet sich hierbei nach den Größen des Kreuzgelenkes 15 bzw. des Zwischengliedes 16. Eine Biegestelle 5 befindet sich hierbei jeweils direkt im Bereich der Lagerstifte 13 eines jeden Einzelgelenkes 15a.

Es wird nicht immer unbedingt erforderlich sein, daß die Wellenteile durch gesonderte Gleitlager im Schaft abgestützt werden, da auch der Schaft direkt Lager für zumindest eines der Wellenteile und/oder Kreuzgelenke bilden kann. Zweckmäßigerweise wird in diesem Fall der Schaft innen mit einer reibungsmindernden Schicht ausgekleidet.

Außerdem kann für den Fall, daß die Wellenteile und Kreuzgelenke hohl bzw. mit einem Durchgang ausgebildet sind, durch diese Durchgänge ein flexibles Rohr vom distalen bis zum proximalen Schaftende geführt werden, welches einen zentral verlaufenden Kanal bildet, über den beispielsweise Spülflüssigkeit, Körpersekrete und dergleichen zugeführt oder abgeführt werden können. Außerdem können durch diesen zentralen Kanal auch Hilfsinstrumente durchgeführt werden, um entsprechende Manipulationen in einer Körperhöhle vornehmen zu können.

Außerdem besteht die Möglichkeit, die Wellenteile exzentrisch im Schaft zu lagern und im größeren Raum zwischen den Wellenteilen und der Schaftinnenwand einen Kanal für Instrumente, Fluide und dergleichen anzuordnen, wobei auch dieser Kanal durch ein starres oder flexibles Rohr gebildet sein kann.

Der Begriff "Biegestelle" besagt allgemein, daß der Instrumentenschaft in Teilabschnitten auf verschiedenen Achsen verläuft. Dabei können die Biegestellen durch Biegen oder Knicken eines Rohres über mehr oder weniger größere Radien geschaffen werden. Möglich ist ist ein Aufbau des Schaftes aus einzelnen Rohrabschnitten, die an ihren jeweils benachbarten Enden durch Löten, Kleben oder dergleichen miteinander verbunden sind. Schließlich kann der Schaft bzw. können die Schäfte auch insgesamt oder nur über einen Teil ihrer Länge beispielsweise im Bereich der Biegestellen flexibel sein, so daß das Instrument wie ein flexibles Endoskop mittels einer proximal vorgesehenen Handhabe gesteuert und mit seinem distalen Ende in die gewünschte Position gebracht werden kann.

## Patentansprüche

1. Instrument (1) zum Abtragen von Gewebe mit einem am distalen Instrumentenende (1a) angeordneten Werkzeug (2), welches über eine Welle (3) antreibbar ist, die durch den starren, mindestens eine Biegestelle (5) aufweisende Schaft (4) des Instrumentes (1) verläuft, dadurch gekennzeichnet, daß die Welle (3) an jeder Biegestelle (5) des Schaftes (4) unterteilt ist, daß die jeweils benachbarten Enden (6a) der starren Wellenteile (6) über mindestens ein Kreuzgelenk (7, 15) in Verbindung stehen und daß jedes Wellenteil (6) im Schaft (4) gelagert ist.

2. Instrument nach Anspruch 1, dadurch gekennzeichnet, daß die Wellenteile (6) über Gleitlager (8) im Schaft (4) gelagert sind.

3. Instrument nach einem der Ansprüche 1 und 2, dadurch gekennzeichnet, daß ein zylindrischer Endbereich (9) des Werkzeuges (2) im Schaft (4) gelagert ist.

4. Instrument nach einem der Ansprüche 1 bis 3, dadurch gekennzeichnet, daß mit Abstand zum vorerwähnten Schaft (4) ein Außenschaft (4a) angeordnet ist und daß der zwischen beiden Schäften (4, 4a) gebildete Raum einen Kanal (10) bildet.

5. Instrument nach einem der Ansprüche 1 bis 4, dadurch gekennzeichnet, daß die Wellenteile (6) und das Kreuzgelenk (7, 15) innen hohl sind.

6. Instrument nach einem der Ansprüche 1 bis 5, dadurch gekennzeichnet, daß das Kreuzgelenk (7) im wesentlichen aus zwei Gelenkgliedern (7a, 7b) besteht, welche mit den Enden (6a) der Wellenteile (6) verbunden sind, derart, daß die Gelenkglieder (7a, 7b) von einer Gelenklasche (12) überbrückt werden, welche die Gelenkglieder (7a, 7b) drehbeweglich über um 90° zueinander versetzt angeordnete Stifte (13) miteinander verbindet, wobei die Stifte (13) in der Gelenklasche (12) fixiert sind und senkrecht zueinander stehende Achsen (14) bilden.

7. Instrument nach einem der Ansprüche 1 bis 6, dadurch gekennzeichnet, daß die jeweils benachbarten Enden (6a) der Wellenteile (6) über ein aus zwei Einzelgelenken (15a) bestehendes doppeltes Kreuzgelenk (15) in Verbindung stehen und daß das Kreuzgelenk (15) ein Zwischenglied (16) aufweist, welches eine Biegestelle (5) überbrückt und die Einzelgelenke verbindet.

8. Instrument nach einem der Ansprüche 1 bis 6, dadurch gekennzeichnet, daß die jeweils benachbarten Enden (6a) der Wellenteile (6) über ein aus zwei Einzelgelenken (15a) bestehendes doppeltes Kreuzgelenk (15) in Verbindung stehen, daß das Kreuzgelenk (15) ein Zwischenglied (16) aufweist, welches die Einzelgelenke verbindet und zwei Biegestellen (5) überbrückt, derart, daß sich jeweils ein Einzelgelenk im Bereich einer Biegestelle befindet.

9. Instrument nach einem der Ansprüche 1 bis 8, dadurch gekennzeichnet, daß der Schaft (4) Lager für zumindest eines der Wellenteile (6) und/oder Kreuzgelenke (7, 15) bildet.

10. Instrument nach Anspruch 9, dadurch gekennzeichnet, daß der Schaft (4) innen mit einer reibungsmindernden Schicht ausgekleidet ist.

11. Instrument nach einem der Ansprüche 1 bis 10, dadurch gekennzeichnet, daß vom distalen bis zum proximalen Schaftende durch die hohl ausgebildeten Wellenteile (6) und Kreuzgelenke (7, 15) ein flexibles Rohr geführt ist.

12. Instrument nach einem der Ansprüche 1 bis 11, dadurch gekennzeichnet, daß die Wellenteile exzentrisch im Schaft gelagert sind und zwischen Wellenteilen und der Schaftinnenwand ein Kanal angeordnet ist.
